(19) Europäisches Patentamt
European Patent Office
Office européen des brevets

(11) Veröffentlichungsnummer: **0 244 355**
**B1**

# (12) EUROPÄISCHE PATENTSCHRIFT

(45) Veröffentlichungstag der Patentschrift: **01.08.90**

(51) Int. Cl.$^5$: **C07C 261/04**, C08K 5/16

(21) Anmeldenummer: **87810248.2**

(22) Anmeldetag: **21.04.87**

## (54) Mit substituierten Aminocarbamaten stabilisierte Zusammensetzungen und neue substituierte Aminocarbamate.

(30) Priorität: **25.04.86 US 855662**

(43) Veröffentlichungstag der Anmeldung: **04.11.87 Patentblatt 87/45**

(45) Bekanntmachung des Hinweises auf die Patenterteilung: **01.08.90 Patentblatt 90/31**

(84) Benannte Vertragsstaaten: **BE CH DE FR GB IT LI NL SE**

(56) Entgegenhaltungen:
**CHEMICAL ABSTRACTS, Band 93, Nr. 19, 10. November 1980, Seite 627, Zusammenfassung Nr. 186006e, Columbus, Ohio, US; & SU-A-727 637 (G.A. SAL'NIKOVA) 15-04-1980**

(73) Patentinhaber: **CIBA-GEIGY AG, Klybeckstrasse 141, CH-4002 Basel(CH)**

(72) Erfinder: **Pastor, Stephen D., 1080 Warburton Avenue Apt. 1C, Yonkers New York 10701(US)**
Erfinder: **Hessell, Edward T., 27 North Taylor Avenue, Norwalk CT 06854(US)**
Erfinder: **Ravichandran, Ramanathan, 111 De Haven Drive Apt. 125, Yonkers New York 10703(US)**

EP 0 244 355 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue mit substituierten Aminocarbamaten stabilisierte Zusammensetzungen, das mit den substituierten Aminocarbamaten gegen oxidativen, thermischen und/oder aktinischen Abbau stabilisierte organische Material und neue substituierte Aminocarbamate.

Polymere organische Materialien, wie Kunststoffe und Harze, sind gegen thermischen, oxidativen und/oder lichtinduzierten Abbau empfindlich. Aus der Literatur sind zahlreiche Verbindungen zum Stabilisieren verschiedener Substrate bekannt. Die Wirksamkeit der Stabilisatoren hängt von den den Abbau bewirkenden Einflüssen und der Art des stabilisierten Substrats ab. Im allgemeinen ist eine Voraussage, welcher Stabilisator auf einem spezifischen Anwendungsgebiet am wirksamsten und wirtschaftlichsten ist, schwierig. So kann z.B. die Stabilisatorwirkung bezüglich Reduktion der Flüchtigkeit davon abhängen, dass in den Substratmolekülen ein Zerfall der Bindungen verhindert wird. Um unter Beibehaltung der Elastizität die Brüchigkeit eines Polymeren oder Kautschuks zu verringern, kann es darauf ankommen, dass eine zu starke Vernetzung und/oder ein Kettenzerfall verhindert werden. Zum Verhindern der Verfärbung können Inhibitorreaktionen, welche im Substrat oder im Stabilisator neue Chromophore oder Farbkörper bilden, erforderlich sein. Zudem müssen auch die Probleme der Verarbeitungsstabilität und der Unverträglichkeit berücksichtigt werden.

Verschiedene organische Hydroxylaminverbindungen sind allgemein bekannt und teilweise im Handel erhältlich. Mehrere Patentschriften offenbaren N-substituierte Hydroxylamine als Antioxidans-Stabilisatoren für verschiedene Substrate, wie Polyolefine, Polyester und Polyurethane. Als repräsentative Beispiele seien die US Patentschriften 3.432.578, 3.644.278, 3.778.464, 3.408.422, 3.926.909, 4.316.996 und 4.386.224 genannt, die im wesentlichen N,N-dialkyl-, N,N-diaryl- und N,N-diaralkyl-substituierte Hydroxylamine und deren farbverbessernde Wirkung und Farbbeständigkeit beschreiben.

In der US Patentschrift 3.869.278 und Pharmazie, 20, 291(1965) [Zinner et al.] sind zudem verschiedene carbamoyl-substituierte Hydroxylamine in anderem Zusammenhang als mit Polymeren beschrieben. Die Umsetzung von N,N-Dimethyl-, N,N-Diethyl- und N,N-Diisopropylhydroxylaminen mit Isocyanaten zur Herstellung von N,N-Dialkyl-O-carbamoylhydroxylaminen mit potentieller herbizider Wirkung wurde von N.V. Konstantinovia et al., in Zhurnal Organicheskoi Khimii, 4, 1928-1932(1968) beschrieben. Ueber die Umsetzung von benzhydryl-substituierten Hydroxylaminen berichteten Wolfgang Kliegel et al. in Liebigs Ann. Chem., 736, 173-175(1970). Das O-Carbamoylhydroxylamin wurde von I.K. Larsen in Acta Chimica Scandinavia, 22, 843-853(1968) beschrieben. Schliesslich wird die Umsetzung von N-Acyl-N-alkylhydroxylaminen mit Isocyanaten von H.G. Aurich et al. im Chem. Ber., 106, 1881-1896(1973) erwähnt. In diesen letzteren Publikationen sind für die erwähnten Verbindungen keine Anwendungen angegeben.

Gegenstand der Erfindung sind Zusammensetzungen, die ein gegen oxidativen, thermischen und/oder aktinischen Abbau empfindliches organisches Material und mindestens eine Verbindung der Formel I

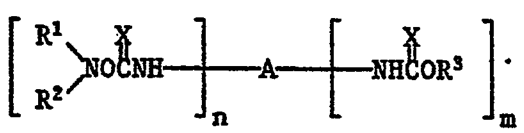

(I)

enthalten, worin

n 1, 2 oder 3 ist, m 0, 1 oder 2 bedeutet und n+m 1, 2 oder 3 darstellen,

$R^1$ und $R^2$ unabhängig voneinander Wasserstoff, $C_1$-$C_{36}$-Alkyl, mit Halogen substituiertes $C_1$-$C_{36}$-Alkyl, $C_2$-$C_6$-Alkoxyalkyl, $C_5$-$C_{12}$-Cycloalykl, $C_7$-$C_9$-Aralkyl oder mit $C_1$-$C_{36}$-Alkyl substituiertes $C_7$-$C_9$-Aralkyl darstellen,

$R^3$ $C_1$-$C_{18}$-Alkyl bedeutet,

X Sauerstoff oder Schwefel ist,

A, bei n = 1 und m = 0, Wasserstoff, $C_1$-$C_{36}$-Alkyl, mit Halogen substituiertes $C_1$-$C_{36}$-Alkyl, $C_2$-$C_6$-Alkoxyalkyl, $C_5$-$C_{12}$-Cycloalkyl, $C_6$-$C_{10}$-Aryl, $C_7$-$C_9$-Aralkyl, durch $C_1$-$C_{36}$-Alkyl substituiertes $C_7$-$C_9$-Aralkyl oder Aryloxyalkyl mit 6 bis 10 C-Atomen im Arylteil und 2-6 C-Atomen im Alkylteil bedeutet,

A, bei n+m = 2, $C_1$-$C_{12}$-Alkylen, $C_2$-$C_{12}$-Alkyliden, $C_6$-$C_{10}$-Cycloalkylen, durch $C_1$-$C_4$-Alkyl substituiertes Hexahydrobenzylen, $C_6$-$C_{10}$-Arylen oder eine Gruppe

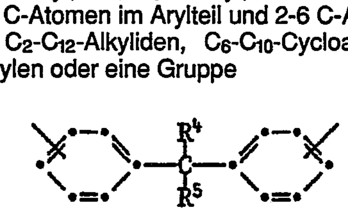

bedeutet, worin $R^4$ und $R^5$ unabhängig voneinander Wasserstoff oder $C_1$-$C_8$-Alkyl darstellen, und

A, bei n+m = 3, $C_3$-$C_{12}$-Alkantriyl oder einen Tris($C_2$-$C_6$-Alkylen)isocyanuratrest darstellt.

Die erfindungsgemässen Zusammensetzungen können auch phenolische Antioxidantien enthalten, wobei die Carbamate der Formel I die durch die Anwesenheit der genannten Phenole bewirkte Farbbildung wesentlich verringern.

Die Aminocarbamate der Formel I verleihen den damit stabilisierten Zusammensetzungen eine Reihe wertvoller Eigenschaften. So können damit verschiedene Substrate, wie z.B. Polyolefine, Elastomere und Schmieröle, gegen den schädigenden Einfluss des oxidativen, thermischen und/oder aktinischen Abbaus geschützt werden. Besonders wirksam sind sie als Farbverbesserer und Verarbeitungsstabilisatoren in Polyolefin-Zusammensetzungen, die gegebenenfalls Metallsalze von Fettsäuren und/oder phenolische Antioxidantien enthalten.

Sie dienen also dazu, die durch die Anwesenheit von phenolischen Antioxidantien und/oder durch die Verarbeitungsbedingungen verursachte Farbbildung merklich zu verringern und auch die Polymeren direkt gegen schädigende Auswirkungen der Verarbeitungsbedingungen zu schützen. Sie verhindern ferner die Verfärbung von Polyolefin-Zusammensetzungen, die sterisch gehinderte Amin-Lichtstabilisatoren oder Kombinationen von phenolischen Antioxidantien und organischen Phosphiten enthalten. Schliesslich wird auch die Verfärbung unter dem Einfluss von Verbrennungsgasen oder natürlichen Gasen merklich reduziert.

$R^1$, $R^2$ und A als $C_1$-$C_{36}$-Alkyl sowie $R^3$ als $C_1$-$C_{18}$-Alkyl können geradkettig oder verzweigt sein und sind beispielsweise Methyl, Ethyl, n-Butyl, 2-Ethylhexyl, n-Octyl, n-Decyl, n-Dodecyl, n-Octadecyl, Icosyl, Tetracosyl, Tricontyl oder Hexatricontyl. Als Alkylgruppen $R^1$, $R^2$ und A werden solche mit 1-18 C-Atomen bevorzugt.

Sind Alkylgruppen $R^1$, $R^2$ oder A durch Halogenatome substituiert, so handelt es sich z.B. um Brom-, Fluor oder Chloratome. Als Beispiele solcher Gruppen seien Chlor- und Brommethyl, Dichlor- und Trifluormethyl, Bromethyl und 2-Chlorbutyl genannt. Beispiele von $C_2$-$C_6$-Alkoxyalkylgruppen $R^1$, $R^2$ oder A sind Methoxymethyl, Ethoxymethyl, 2-Methoxyethyl, 2-Ethoxyethyl und 3-Methoxypropyl.

$R^1$, $R^2$ und A als $C_5$-$C_{12}$-Cycloalkyl sind beispeilsweise Cyclopentyl, Cyclohexyl, Cyclooctyl oder Cyclododecyl. Bevorzugt ist Cycloalkyl mit 5 bis 7 "-$CH_2$-"-Ringgliedern. Besonders bevorzugt ist Cyclohexyl.

$R^1$, $R^2$ und A können als unsubstituiertes oder durch $C_1$-$C_{36}$-Alkyl substituiertes $C_7$-$C_9$-Aralkyl insbesondere Phenylalkyl mit 7 bis 9 Kohlenstoffatomen bedeuten, wobei der Phenylring gegebenenfalls durch $C_1$-$C_{36}$-Alkyl, bevorzugt Methyl, substituiert sein kann. Als Beispiele seien genannt: Benzyl, α-Methylbenzyl, α,α-Dimethylbenzyl, 3- oder 4-Methylbenzyl, 3,5-Dimethylbenzyl oder 4-Nonylbenzyl. Bevorzugt sind α-Methylbenzyl, α,α-Dimethylbenzyl und vor allem Benzyl.

Stellt A $C_6$-$C_{10}$-Aryl dar, so handelt es sich z.B. um unsubstituiertes oder durch $C_1$-$C_4$-Alkylgruppen, besonders Methylgruppen, substituiertes Phenyl oder um unsubstituiertes Naphthyl. Unsubstituiertes Phenyl ist bevorzugt.

Ist A Aryloxyalkyl mit 6-10 C-Atomen im Arylteil und 2-6 C-Atomen im Alkylteil, so handelt es sich insbesondere um Phenoxyalkylgruppen, wie z.B. Phenoxyethyl-, Phenoxypropyl- und Phenoxybutylgruppen.

Alkylengruppen A können geradkettig oder verzweigt sein und stellen zum Beispiel Ethylen, 1,2-Propylen, Trimethylen, Tetramethylen, Hexamethylen, Octamethylen oder Dodecamethylen dar. Besonders bevorzugt ist Hexamethylen.

Alkylidengruppen A(n+m = 2) können geradkettig oder verzweigt sein und weisen bevorzugt 2-6 C-Atome auf. Als Beispiele seien erwähnt: Ethyliden, Propyliden, Isopropyliden, Butyliden, Hexyliden und Octyliden. Bevorzugt ist Ethyliden.

Als $C_6$-$C_{10}$-Cycloalkylen stellt A beispielsweise Cyclohexylen, Cyclooctylen oder Cyclodecylen dar. Bevorzugt ist Cyclohexylen.

Bedeutet A durch $C_1$-$C_4$-Alkyl substituiertes Hexahydrobenzylen, so kann der Hexahydrobenzylenrest durch eine oder mehrere Alkylgruppen, besonders Methylgruppen, substituiert sein. Insbesondere handelt es sich dabei um Trimethylhexahydrobenzylen, vor allem das 3-Methylen-3,5,5-trimethylcyclohexylen-1.

$C_6$-$C_{10}$-Arylengruppen A können unsubstituiert sein substituiert sein, besonders durch $C_1$-$C_4$-Alkylgruppen, vor allem Methylgruppen. Als Beispiele solcher Reste seien 1,2-, 1,3- und 1,4-Phenylen, 1,4-, 1,6- und 2,6-Naphthylen und 2,4-Tolylen genannt. 1,4-Phenylen und besonders 2,4-Tolylen sind bevorzugt. Stellt A Xylylen dar, so handelt es sich z.B. um o-Xylylen und vor allem um m-Xylylen.

Alkylgruppen $R^4$ und $R^5$ in Resten

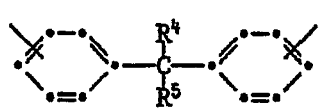

können geradkettig oder verzweigt sein. Bevorzugt sind geradkettige $C_1$-$C_4$-Alkylgruppen. Besonders bevorzugt stellen $R^4$ und $R^5$ Wasserstoff dar.

Stellt A $C_3$-$C_{12}$-Alkantriyl dar, so handelt es sich beispielsweise um Propan-1,2,3-triyl, Hexan-1,2,6-triyl oder den Rest

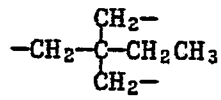

Bedeutet A einen Tris($C_2$-$C_6$-Alkylen)isocyanuratrest, so kann das Alyklen geradkettig oder verzweigt sein. Bevorzugt sind geradkettige Alkylengruppen.

$R^1$ und $R^2$ sind bevorzugt unabhängig voneinander Wasserstoff oder geradkettiges oder verzweigtes Alkyl mit 1-18 C-Atomen, wie Methyl, Ethyl, n-Propyl, n-Butyl, tert-Butyl, n-Pentyl, n-Octyl, 2-Ethylhexyl, n-Decyl, n-Dodecyl und n-Octadecyl; Cyclopentyl, Cyclohexyl, Benzyl, α-Methylbenzyl oder α,α-Dimethylbenzyl. Besonders bevorzugt stellen $R^1$ und $R^2$ je Benzyl dar.

X ist vorzugsweise 0. $R^3$ ist bevorzugt $C_{12}$-$C_{18}$-Alkyl, vor allem n-Octadecyl.

Bei n = 1 und m = 0 stellt A vorzugsweise geradkettiges oder verzweigtes Alkyl mit 1-18 C-Atomen oder Phenyl dar. Beispiele solcher Alkylgruppen sind unter $R^1$ und $R^2$ genannt. Bei n+m = 2 [n und m = 1 oder n = 2 und m = 0] bedeutet A vorzugsweise $C_2$-$C_6$-Alkylen, wie Ethylen, Propylen und Hexylen, Alkyliden mit 2-6 C-Atomen, besonders Ethyliden, Cyclohexylen, Trimethyl-hexahydrobenzylen, Phenylen, 2,4-Tolylen, Xylylen oder den 4,4-Diphenylmethanrest. Besonders bevorzugt sind Hexamethylen, 2,4-Tolylen und 3-Methylen-3,5,5-trimethylcyclohexylen-1. Bei n+m = 3 [n = 1 und m = 2, n = 2 und m = 1 oder n = 3 und m = 0] stellt A bevorzugt

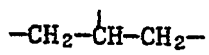 oder 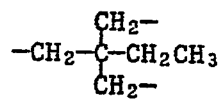

und insbesondere den Rest des Tris(hexylen)isocyanurats dar; m ist bevorzugt 0 oder 1.

Gegenstand der Erfindung sind auch die neuen Verbindungen der Formel Ia

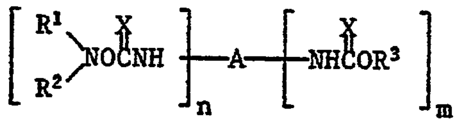 (Ia),

worin n 1, 2 oder 3, m 0, 1 oder 2 und n+m 2 oder 3 sind, und $R^1$, $R^2$, $R^3$, X und A (für n+m = 2 oder 3) die unter Formel I angegebene Bedeutung haben. Dabei gilt für bevorzugte Bedeutungen von n, m, $R^1$, $R^2$, $R^3$, X und A das oben Angegebene.

Die Verbindungen der Formel I und Ia können auf an sich bekannte Weise z.B. dadurch hergestellt werden, dass man ein Hydroxylamin der Formel II

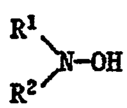 (II)

in Gegenwart eines Lösungsmittels mit einem Cyanatsalz (A = H) oder einer Verbindung der Formel III

$$A\text{-}[\text{-}N{=}C{=}X]_n \quad (III)$$

umsetzt und das erhaltene Reaktionsprodukt gegebenenfalls anschliessend mit einem Alkohol der Formel IV

$$R^3\text{-}OH \quad (IV)$$

(m = 1 oder 2) umsetzt, wobei $R^1$, $R^2$, $R^3$, X, A und n die unter Formel I angegebene Bedeutung haben. Geeignete Cyanatsalze und Verbindungen der Formel III sind z.B. Alkalimetallcyanate, wie Na- und K-Cyanat, n-Butylisocyanat, n-Hexylisocyanat, n-Octadecylisocyanat, Phenylisocyanat, 2,4-Tolylendiisocyanat, 1,6-Hexandiisocyanat, Ethylisothiocyanat, Phenylisothiocyanat und Naphthylisothiocyanat. Als Lösungsmittel, besonders bei A ≠ H, eignen sich beispielsweise aromatische Kohlenwasserstoffe, wie Benzol, Toluol und Xylole, oder heterocyclische Ether, wie Dioxan und vor allem Tetrahydrofuran. Die Umsetzung der Hydroxylamine mit den Cyanatsalzen wird üblicherweise in wässrig-alkoholischem Medium, z.B. in einem Gemisch aus Methanol und Wasser, durchgeführt. Die Reaktionstemperaturen für die Umsetzung der Hydroxylamine mit den Verbindungen der Formel III bzw. den Cyanatsalzen liegen zweckmässig zwischen 10 und 70°C. Die allfällige weitere Umsetzung des so erhaltenen Reaktionsprodukts mit einem Alkohol $R^3$-OH wird bevorzugt bei Temperaturen zwischen 20 und 70°C vorgenommen. Die Aus-

gangsprodukte der Formeln II, III und IV sowie die Cyanatsalze sind im Handel erhältlich oder können auf an sich bekannte Weise hergestellt werden.

Die Verbindungen der Formel I eignen sich besonders zum Stabilisieren von gegen oxidativen, thermischen und/oder aktinischen Abbau empfindlichen organischen Materialien, wie Kunststoffe, Polymere und Harze.

Substrate, die sich mit den Verbindungen der Formel I besonders gut stabilisieren lassen sind z.B. Polyolefine, wie Polyethylen und Polypropylen, Polystyrol einschliesslich schlagfestem Polystyrol, ABS-Harze, SBR-Harze, Isopren, natürliche Kautschuke, Polyester, wie z.B. Polyethylenterephthalat- und Polybutylenterephthalat-homo- und -copolymere. Schmieröle, z.B. solche auf Basis von Mineralölen.

Als Beispiele geeigneter Substrate seien genannt:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vertnetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, dis Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator z.B. Poly-2,4,4-trimethylhexamethylenterephthalamid, Polym-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalt, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Das organische Material ist vorzugsweise ein synthetisches Polymer, insbesondere ein Polyolefinhomopolymer oder -copolymer.

Die Verbindungen der Formel I werden im allgemeinen in Mengen von etwa 0,01 bis etwa 5 Gew.%, bezogen auf die stabilisierte Zusammensetzung, eingesetzt, wobei die eingesetzte Menge von der Art des verwendeten Substrats und der gewünschten Anwendung abhängt. Bevorzugt verwendet man die Verbindungen der Formel I in einer Menge von etwa 0,5 bis etwa 2 Gew.%, insbesondere etwa 0,1 bis etwa 1 Gew.%, bezogen auf die stabilisierte Zusammensetzung.

Die Einarbeitung der Verbindungen der Formel I in das zu stabilisierende organische Material kann nach bekannten Methoden während jeder Verarbeitungsstufe vor der Formgebung erfolgen. Der Stabilisator kann z.B. den zu stabilisierenden Materialien in Pulverform zugemischt werden, oder eine Suspension oder Emulsion des Stabilisators kann mit einer Lösung, Suspension oder Emulsion des zu stabilisierenden Materials vermischt werden. Die so stabilisierten Materialien können in verschiedenster

Form angewendet werden, z.B., als Folien, Fasern, Bändchen, Formmassen, Profile oder als Bindemittel für Lacke, Klebstoffe oder Kitte.

Die erfindungsgemässen stabilisierten Zusammensetzungen können gegebenenfalls auch übliche Additive enthalten.

Beispiele für herkömmliche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.-butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4- hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Bis-(3-tert.butyl-4-hydroxy-5-methylphenyl)-dicyclopentadien, Bis-[2-(3'-tert.butyl-2'-hydroxy-5'-methyl-benzyl)-6-tert.butyl-4-methylphenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylestr, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxy-stearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der ß-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der ß-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)-ethyl-isocyanurat, N,N'-Bis-(hydroxyethyl)-oxalsäurediamid.

1.9. Ester der ß-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der ß-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.-butylphenylester, 3,5-Di-tert.butyl-4-hydroxybenzoesäurehexadecylester.

2.4. Acrylate, wie z.B. α-Cyan-ß,ß-diphenylacrylsäure-ethylester bzw. -isooctylester, α-Carbomethoxy-zimtsäuremethylester, α-Cyano-ß-methyl-p-methoxy-zimtsäuremethylester bzw. -butylester, α-Carbomethoxy-p-methoxy-zimtsäure-methylester, N-(ß-Carbomethoxy-ß-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyl-dithiocarbamat, Nickelsalze von 4-Hydroxy-3,5-di-tert.butylbenzyl-phosphonsäure-monoalkylestern, wie vom Methyl- oder Ethylester, Nickelkomplexe von Ketoximen, wie von 2-Hydroxy-4-methyl-phenyl-undecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-di-methylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der ß-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(ß-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Bevorzugt enthalten die erfindungsgemässen Zusammensetzungen als weitere Zusätze ein Metallsalz einer höheren Fettsäure und/oder ein phenolisches Antioxidans.

Obschon sich die Aminocarbamate der Formel I allein oder in Mischung mit anderen Additiven zum Stabilisieren verschiedenartiger Substrate, besonders Polyolefine, eignen, bewirkt der Zusatz dieser Stabilisatoren zusammen mit sterisch gehinderten phenolischen Antioxidantien zu verschiedenen Zusammensetzungen, besonders Polyolefinen, die gegebenenfalls Alkalimetall-, Erdalkalimetall- und/oder Aluminiumsalze von höheren Fettsäuren enthalten (vgl. Punkt 7 oben) einen besonders guten Schutz der Substrate hinsichtlich der in Gegenwart der genannten Phenole auftretenden Farbbildung. Geeignete phenolische Antioxidantien sind:

n-Octadecyl-(3,5-di-tert-butyl-4-hydroxyhydrocinnamat),
Neopentantetrayl-tetrakis[3,5-di-tert-butyl-4-hydroxyhydrocinnamat],
Di-n-octadecyl-(3,5-di-tert-butyl-4-hydroxybenzyl)phosphonat,
1,3,5-Tris[3,5-di-tert-butyl-4-hydroxybenzyl]isocyanurat,
Thiodiethylen-bis[3,5-di-tert-butyl-4-hydroxyhydrocinnamat],
1,3,5-Trimethyl-2,4,6-tris[3,5-di-tert-butyl-4-hydroxybenzyl]benzol,
3,6-Dioxaoctamethylen-bis[3-methyl-5-tert-butyl-4-hydroxyhydrocinnamat],

2,6-Di-tert-butyl-p-kresol,
2,2'-Ethyliden-bis[4,6-di-tert-butyl-phenol],
1,3,5-Tris[2,6-dimethyl-4-tert-butyl-3-hydroxybenzyl]isocyanurat,
1,1,3-Tris[2-methyl-4-hydroxy-5-tert-butylphenyl]butan,
1,3,5-Tris[2-(3,5-di-tert-butyl-4-hydroxyhydrocinnamoyloxy)-ethyl]isocyanurat,
3,5-Bis[3,5-di-tert-butyl-4-hydroxybenzyl]mesitol,
Hexamethylen-bis[3,5-di-tert-butyl-4-hydroxyhydrocinnamat],
1-(3,5-Di-tert-butyl-4-hydroxyanilino)-3,5-dioctylthio-s-triazin,
N,N'-Hexamethylen-bis[3,5-di-tert-butyl-4-hydroxyhydrocinnamamid],
Kalzium-Bis[ethyl-(3,5-di-tert-butyl-4-hydroxybenzyl)phosphonat],
Ethylen-bis[3,3-bis(3-tert-butyl-4-hydroxyphenyl)butyrat],
Octyl-(3,5-di-tert-butyl-4-hydroxybenzylmercaptoacetat),
Bis[3,5-di-tert-butyl-4-hydroxyhydrocinnamoyl]hydrazid und
N,N'-bis[2-(3,5-di-tert-butyl-4-hydroxyhydrocinnamoyloxy)ethyl]oxamid.

Besonders bevorzugt sind
Neopentantetrayl-tetrakis[3,5-di-tert-butyl-4-hydroxyhydrocinnamat],
n-Octadecyl-(3,5-di-tert-butyl-4-hydroxyhydrocinnamat),
1,3,5-Trimethyl-2,4,6-tris[3,5-di-tert-butyl-4-hydroxybenzyl]benzol,
1,3,5-Tris[3,5-di-tert-butyl-4-hydroxybenzyl]isocyanurat,
2,6-Di-tert-butyl-p-kresol und
2,2'-Ethyliden-bis[4,6-di-tert-butyl-phenol].

Die Verbindungen der Formel I verhindern in Gegenwart sterisch gehinderter Amin-Lichtstabilisatoren auch Verfärbungen, z.B. in Gegenwart von Bis(1,2,2,6,6-Pentamethyl-4-piperidyl)-2-n-butyl-2-(3,5-di-tert-butyl-4-hydroxybenzyl)malonat, Bis(2,2,6,6-Tetramethyl-4-piperidyl)sebacat, Dimethylsuccinat-Polymer mit 4-Hydroxy-2,2,6,6-tetramethyl-1-piperidinethanol und Polymeren aus 2,4-Dichlor-6-tert-octylamino-s-triazin und N,N'-(2,2,6,6-Tetramethyl-4-piperidyl)hexamethylendiamin.

Die folgenden Beispiele erläutern die Erfindung weiter. Alle Mengenangaben beziehen sich auf das Gewicht, sofern nichts anderes angegeben ist.

Beispiel 1: Herstellung von N-n-Butyl-O-(N,N-dibenzylamino)carbamat

Eine Lösung von 5,33 g (25 mmol) N,N-Dibenzylhydroxylamin in 25 ml wasserfreiem Tetrahydrofuran wird mit 2,48 g (25 mmol) n-Butylisocyanat vermischt, und das Gemisch wird während 4 Std. gerührt. Das Lösungsmittel wird im Vakuum entfernt und der Rückstand wird aus Heptan umkristallisiert. Man erhält 6,8 g (87 %d.Th.) des N-n-Butyl-O-(N,N-dibenzylamino)carbamats in Form eines weissen Feststoffes; Smp. 81-82°C.
Analyse für $C_{19}H_{24}N_2O_2$:
berechnet C 73,0; H 7,7; N 9,0
gefunden C 73,0; H 7,7; N 8,8.

Beispiel 2: Herstellung von N-Phenyl-O-(N,N-dibenzylamino)carbamat

Das in Beispiel 1 beschriebene Verfahren wird wiederholt unter Verwendung von 5,33 g (25 mmol) N,N-Dibenzylhydroxylamin und 2,98 g (25 mmol) Phenylisocyanat. Der Rückstand wird aus einem Heptan-Toluol-Lösungsmittelgemisch umkristallisiert, wobei 5,8 g (70 % d.Th.) des N-Phenyl-O-(N,N-dibenzylamino)carbamats in Form eines weissen Feststoffes erhalten werden; Smp. 120-123°C.
Analyse für $C_{21}H_{20}N_2O_2$:
berechnet C 75,9; H 6,1; N 8,4
gefunden C 75,6; H 5,9; N 8,3.

Beispiel 3: Herstellung von N-n-Octadecyl-O-(N,N-dibenzylamino)-carbamat

Das in Beispiel 1 beschriebene Verfahren wird wiederholt unter Verwendung von 5,33 g (25 mmol) N,N-Dibenzylhydroxylamin und 7,38 g (12,5 mmol) n-Octadecylisocyanat. Nach dem Umkristallisieren des Rückstands aus Heptan erhält man 9,0 g (71 % d.Th.) N-n-Octadecyl-O-(N,N-dibenzylamino)carbamat in Form eines weissen Feststoffes; Smp. 63-66°C.
Analyse für $C_{33}H_{52}N_2O_2$:
berechnet C 77,9; H 10,3; N 5,5
gefunden C 78,5; H 10,6; N 5,7.

Beispiel 4: Herstellung von N,N'-(1,6-Hexandiyl)-bis[O-(N,N-dibenzylamino)carbamat]

Das Verfahren gemäss Beispiel 1 wird wiederholt, unter Verwendung von 5,33 g (25 mmol) N,N-Dibenzylhydroxylamin und 2,10 g (1,25 mmol) 1,6-Hexandiisocyanat. Nach dem Umkristallisieren des Rückstands aus 2-Propanol erhält man 6,6 g (89 % d.Th.) des N,N'-(1,6-Hexandiyl)-bis[O-(N,N-dibenzylamino)carbamats] in Form eines weissen Feststoffes; Smp. 121-124°C.
Analyse für $C_{36}H_{42}N_4O_4$:
berechnet C 72,7; H 7,1; N 9,3
gefunden C 72,6; H 7,1; N 9,4.

Beispiel 5: Herstellung von N,N'-(4-Methyl-1,3-phenylen)-di[O-N,N-dibenzylamino)carbamat]

Das Verfahren gemäss Beispiel 1 wird wiederholt, unter Verwendung von 42,66 g (0,2 Mol) N,N-Dibenzylhydroxylamin und 17,42 g (0,1 Mol) 2,4-Toluoldiisocyanat. Man erhält 57,0 g des N,N'-(4-Methyl-1,3-phenylen)-di[O-(N,N-dibenzylamino)carbamats] in Form eines weissen Feststoffes; Smp. 48-55°C.
Analyse für $C_{37}H_{36}N_4O_4$:
berechnet C 74,0; H 6,0; N 9,3
gefunden C 73,6; H 6,1; N 9,1.

Beispiel 6: Herstellung von N,N'-(4-Methyl-1,3-phenylen)-O-(N,N-dibenzylamino)-O-(n-octa decyl)dicarbamat

Ein Gemisch aus 21,33 g (0,1 Mol) N,N-Dibenzylhydroxylamin in 100 ml Tetrahydrofuran wird unter Rühren tropfenweise mit 17,42 g (0,1 Mol) 2,4-Toluoldiisocyanat versetzt. Das Reaktionsgemisch wird bis zur Beendigung der Umsetzung, ermittelt durch das Verschwinden der Hydroxylabsorption im IR-Spektrum des Reaktionsgemisches, bei Raumtemperatur gerührt. Das erhaltene Reaktionsgemisch wird mit 27,05 g (0,1 Mol) n-Octadecylalkohol versetzt und dann während 14 Std. zum Rückfluss erhitzt. Nach dem Abkühlen wird der entstandene Feststoff durch Filtrieren abgetrennt und zweimal aus Aceton umkristallisiert. Man erhält 12,31 g der oben genannten Verbindung als weissen Feststoff; Smp. 90-97°C.

Beispiel 7: Herstellung von N,N-Dibenzyl-O-carbamoylhydroxylamin

Zu einer Suspension von 10,70 g N,N-Dibenzylhydroxylamin in 25 ml Methanol wird unter Rühren tropfenweise eine Lösung von 4,53 ml konzentrierter HCl in 10 ml Wasser gegeben. Dann gibt man eine Lösung von 4,06 g Kaliumcyanat in 15 ml Wasser zu und rührt die entstandene Suspension während 3 Std. bei Raumtemperatur. Das Rohprodukt wird durch Filtration abgetrennt und mittels Flüssigchromatographie gereinigt. Man erhält das N,N-Dibenzyl-O-carbamoylhydroxylamin in Form eines weissen Feststoffes; Smp. 127-129°C.
Analyse für $C_{15}H_{16}N_2O_2$:
berechnet C 70,3; H 6,3; N 10,9
gefunden C 70,2; H 6,3; N 11,2.

Beispiel 8: Herstellung von N-n-Hexyl-O-(N,N-dibenzylamino)carbamat

Das Verfahren gemäss Beispiel 1 wird wiederholt unter Verwendung von 5,33 g (25 mmol) N,N-Dibenzylhydroxylamin und 3,18 g (25 mmol) n-Hexylisocyanat. Nach dem Umkristallisieren des Rückstands aus Heptan erhält man 7,5 g (88 % d.Th.)
N-n-Hexyl-O-(N,N-dibenzylamino)carbamat) in Form eines weissen Feststoffes; Smp. 72-73,5°C.
Analyse für $C_{21}H_{28}N_2O_2$:
berechnet C 74,l; H 8,3; N 8,2
gefunden C 74,4; H 8,2; N 8,2.

Beispiel 9: Herstellung von 1-{N-[O-(N,N-Dibenzylamino)carboxyl]amino}-3-{N-[O-(N,N-dibenzylamino)carboxyl]amino}methyl-3,5,5-trimethylcyclohexan

Das Verfahren gemäss Beispiel 1 wird wiederholt unter Verwendung von 10,66 g (50 mmol) N,N-Dibenzylhydroxylamin und 5,56 g (25 mmol) 3-Isocyanatomethyl-3,5,5-trimethylcyclohexylisocyanat. Nach dem Umkristallisieren des Rückstands aus einem 1:1-Gemisch aus Heptan und Toluol erhält man 11,0 g (68 % d.Th.) des obigen Produkts in Form eines weissen Feststoffes; Smp. 139-146°C.
Analyse für $C_{40}H_{48}N_4O_4$:
berechnet C 74,0; H 7,5; N 8,6
gefunden C 74,0; H 7,7; N 8,7.

Beispiel 10: Herstellung eines Kondensationsproduktes aus N,N-Dibenzylhydroxylamin und N,N',N''-Tris(6-isocyanatohexyl)-2,4,6-trioxo-1,3,5-cyclohexyltriazin

Das Verfahren gemäss Beispiel 1 wird wiederholt unter Verwendung von 5,33 g (25 mmol) N,N-Dibenzylhydroxylamin und 4,66 g einer 90%-igen Lösung von N,N',N''-Tris(6-isocyanatohexyl)-2,4,6-trioxo-1,3,5-cyclohexyltriazin in n-Butylacetat. Der erhaltene Rückstand (8,7 g, 92 % d.Th.) muss nicht weiter gereinigt werden.
Analyse für $C_{66}H_{81}N_9O_9$:
berechnet C 69,3; H 7,1
gefunden C 69,4; H 7,1.

Beispiel: Verarbeitung von Polypropylen

Die Grundformulierung enthält 100 Teile Polypropylen (Profax 6501® der Fa. Himont, USA) und 0,1 Teil Kalziumstearat. Die in der folgenden Tabelle angegebenen Stabilisatoren werden in Form von Lösungen in Methylenchlorid mit dem Polypropylen vermischt. Nach dem Verdampfen des Lösungsmittels unter vermindertem Druck wird das Harz bei den folgenden Bedingungen bei 100 Umdrehungen/Minute extrudiert:

Extrusionsbedingungen

Zylinder 1 232°C Düse 1 260°C;
Zylinder 2 246°C Düse 2 260°C;
Zylinder 3 260°C Düse 3 260°C.

Nach der ersten, dritten und fünften Extrusion werden die Harzkügelchen unter Druck bei 193°C zu 3,2 mm dicken Folien verformt. Der "Yellowness Index" der Proben wird gemäss ASTM D 1925-63T bestimmt. Die Schmelzfliessgeschwindigkeit wird gemäss ASTM-1238, Bedingungen L, bestimmt. Höhere Werte bedeuten geringere Molmasse und sind ein Hiniweis darauf, dass sich das Polymer abgebaut hat.

Tabelle

| Stabilisator | Extrusionstemperatur 260°C | | | | |
|---|---|---|---|---|---|
| | Yellowness Index nach | | | Schmelzfliessgeschw. g/10 Min. nach | |
| | 1.Extrusion | 3.Extrusion | 5.Extrus. | 1.Extrusion | 5.Extrusion |
| ohne | 3,6 | 3,9 | 4,6 | 4,4 | 11,5 |
| 0,1 % Antioxidans A | 6,1 | 7,9 | 9,4 | 2,5 | 4,2 |
| 0,1 % Antioxidans A + 0,05 % Bsp. 1 | 2,9 | 3,4 | 6,5 | 2,2 | 4,1 |
| 0,1 % Antioxidans A + 0,05 % Bsp. 2 | 3,9 | 5,4 | 7,4 | 1,9 | 3,8 |

Antioxidans A = Neopentyl-tetrakis[3-(3',5'-di-tert.butyl-4-hydroxyphenyl)propanoat].

## Patentansprüche

1. Zusammensetzungen enthaltend ein gegen oxidativen, thermischen und/oder aktinischen Abbau empfindliches organisches Material und mindestens eine Verbindung der Formel I

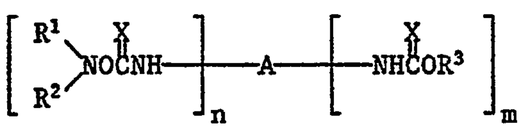

(I),

worin
n 1, 2 oder 3 ist, m 0, 1 oder 2 bedeutet und n+m 1, 2 oder 3 darstellen,

$R^1$ und $R^2$ unabhängig voneinander Wasserstoff, $C_1$-$C_{36}$-Alkyl, mit Halogen substituiertes $C_1$-$C_{36}$-Alkyl, $C_2$-$C_6$-Alkoxyalkyl, $C_5$-$C_{12}$-Cycloalkyl, $C_7$-$C_9$-Aralkyl oder mit $C_1$-$C_{36}$-Alkyl substituiertes $C_7$-$C_9$-Aralkyl darstellen,
$R^3$ $C_1$-$C_{18}$-Alkyl bedeutet,
X Sauerstoff oder Schwefel ist,
A, bei n = 1 und m = 0, Wasserstoff, $C_1$-$C_{36}$-Alkyl, mit Halogen substituiertes $C_1$-$C_{36}$-Alkyl, $C_2$-$C_6$-Alkoxyalkyl, $C_5$-$C_{12}$-Cycloalkyl, $C_6$-$C_{10}$-Aryl, $C_7$-$C_9$-Aralkyl, durch $C_1$-$C_{36}$-Alkyl substituiertes $C_7$-$C_9$-Aralkyl oder Aryloxyalkyl mit 6 bis 10 C-Atomen im Arylteil und 2-6 C-Atomen im Alkylteil bedeutet,
A, bei n+m = 2, $C_1$-$C_{12}$-Alkylen, $C_2$-$C_{12}$-Alkyliden, $C_6$-$C_{10}$-Cycloalkylen, durch $C_1$-$C_4$-Alkyl substituiertes Hexahydrobenzylen, $C_6$-$C_{10}$-Arylen, Xylylen oder eine Gruppe

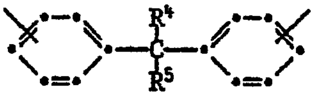

bedeutet, worin $R^4$ und $R^5$ unabhängig voneinander Wasserstoff oder $C_1$-$C_8$-Alkyl darstellen, und
A, bei n+m = 3, $C_3$-$C_{12}$-Alkantriyl oder einen Tris($C_2$-$C_6$-Alkylen)isocyanuratrest darstellt.

2. Zusammensetzung nach Anspruch 1, worin $R^1$ und $R^2$ unabhängig voneinander Wasserstoff, geradkettiges oder verzweigtes Alkyl mit 1-18 C-Atomen, Cyclopentyl, Cyclohexyl, Benzyl, α-Methylbenzyl oder α,α-Dimethylbenzyl darstellen.

3. Zusammensetzung nach Anspruch 1, worin $R^1$ und $R^2$ je Benzyl darstellen.

4. Zusammensetzung nach Anspruch 1, worin X Sauerstoff darstellt.

5. Zusammensetzung nach Anspruch 1, worin n 1 ist, m 0 darstellt und A $C_1$-$C_{18}$-Alkyl oder Phenyl bedeutet.

6. Zusammensetzung nach Anspruch 1, worin n und m je 1 sind oder n 2 ist und m 0 ist und A Alkylen mit 2-6 C-Atomen, Alkyliden mit 2-6 C-Atomen, Cyclohexylen, Trimethyl-hexahydrobenzylen, Phenylen, Xylylen, 2,4-Tolylen oder den 4,4'-Diphenylmethanrest darstellt.

7. Zusammensetzung nach Anspruch 1, worin n+m = 3 ist und A

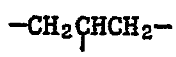 , 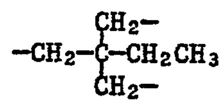

oder den Tris(hexylen)isocyanuratrest bedeutet.

8. Zusammensetzung nach Anspruch 1, worin das organische Material ein synthetisches Polymer ist.

9. Zusammensetzung nach Anspruch 8, worin das synthetische Polymer ein Polyolefinhomo- oder -copolymer ist.

10. Zusammensetzung nach Anspruch 1, die zudem ein Metallsalz einer höheren Fettsäure und/oder ein phenolisches Antioxidans enthält.

11. Verwendung von Verbindungen der Formel I nach Anspruch 1 zum Stabilisieren von gegen oxidativen, thermischen und/oder aktinischen Abbau empfindlichen organischen Materialien.

12. Verbindungen der Formel Ia

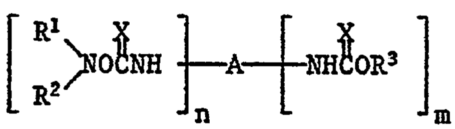

(Ia),

worin
n 1, 2 oder 3 ist, m 0, 1 oder 2 ist und n+m 2 oder 3 sind,
$R^1$ und $R^2$ unabhängig voneinander Wasserstoff, $C_1$-$C_{36}$-Alkyl, mit Halogen substituiertes $C_1$-$C_{36}$-Alkyl, $C_2$-$C_6$-Alkloxyalkyl, $C_5$-$C_{12}$-Cycloalkyl, $C_7$-$C_9$-Aralkyl oder mit $C_1$-$C_{36}$-Alkyl substituiertes $C_7$-$C_9$-Aralkyl darstellen,
$R^3$ $C_1$-$C_{18}$-Alkyl bedeutet,
X Sauerstoff oder Schwefel ist,
A, bei n+m = 2, $C_1$-$C_{12}$-Alkylen, $C_2$-$C_{12}$-Alkyliden, $C_6$-$C_{10}$-Cycloalkylen, durch $C_1$-$C_4$-Alkyl substituiertes Hexahydrobenzylen, $C_6$-$C_{10}$-Arylen, Xylylen oder eine Gruppe

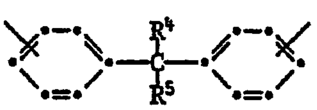

bedeutet, worin $R^4$ und $R^5$ unabhängig voneinander Wasserstoff oder $C_1$-$C_8$-Alkyl darstellen, und A, bei n+m = 3, $C_3$-$C_{12}$-Alkantriyl oder einen Tris($C_2$-$C_6$-Alkylen)isocyanuratrest darstellt.

13. N,N'-(1,6-Hexandiyl)-bis[O-(N,N-dibenzylamino)carbamat], N,N'-(4-Methyl-1,3-phenylen)-di[O-(N,N-dibenzylamino)-carbamat], N,N'-(4-Methyl-1,3-phenylen)-O-(N,N-dibenzylamino)-O-(n-octadecyl)-dicarbamat, 1-{N-[O-(N,N-dibenzylamino)-carboxy]amino}-3-{N-[O-(N,N-dibenzylamino)carboxy]amino}-methyl-3,5,5-trimethylcyclohexan und das Kondensationsprodukt aus N,N-Dibenzylhydroxylamin und N,N', N"-Tris(6-isocyanatohexyl)-2,4,6-trioxo-1,3,5-cyclohexyltriazin nach Anspruch 12.

## Claims

1. A composition containing an organic material subject to oxidative, thermal and/or actinic degradation and at least one compound of the formula I

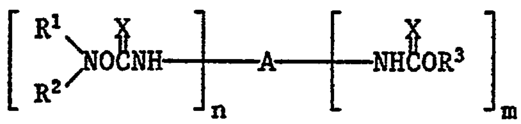 (I),

in which
n is 1, 2 or 3, m is 0, 1 or 2 and n + m is 1, 2 or 3,
$R^1$ and $R^2$ independently of one another are hydrogen, $C_1$–$C_{36}$alkyl, halogen-substituted $C_1$–$C_{36}$alkyl, $C_2$–$C_6$alkoxyalkyl, $C_5$–$C_{12}$cycloalkyl, $C_7$–$C_9$aralkyl or $C_1$–$C_{36}$alkyl-substituted $C_7$–$C_9$aralkyl,
$R^3$ is $C_1$–$C_{18}$alkyl,
X is oxygen or sulfur,
A, when n = 1 and m = 0, is hydrogen, $C_1$–$C_{36}$alkyl, halogen-substituted $C_1$–$C_{36}$alkyl, $C_2$–$C_6$alkoxyalkyl, $C_5$–$C_{12}$cycloalkyl, $C_6$–$C_{10}$aryl, $C_7$–$C_9$aralkyl, $C_1$–$C_{36}$alkyl-substituted $C_7$–$C_9$aralkyl or aryloxyalkyl having 6 to 10 carbon atoms in the aryl moiety and 2 to 6 carbon atoms in the alkyl moiety,
A, when n + m = 2, is $C_1$–$C_{12}$alkylene, $C_2$–$C_{12}$alkylidene, $C_6$–$C_{10}$cycloalkylene, $C_1$–$C_4$alkyl-substituted hexahydrobenzylene, $C_6$–$C_{10}$arylene, xylylene or a group

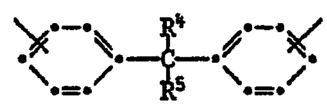

in which $R^4$ and $R^5$ independently of one another are hydrogen or $C_1$–$C_8$alkyl, and
A, when n + m = 3, is $C_3$–$C_{12}$alkanetriyl or a tris($C_2$–$C_6$alkylene)isocyanurate radical.

2. A composition according to claim 1, in which $R^1$ and $R^2$ independently of one another are hydrogen, straight-chain or branched alkyl of 1 to 18 carbon atoms, cyclopentyl, cyclohexyl, benzyln, α-methylbenzyl or α,α-dimethylbenzyl.

3. A composition according to claim 1, in which $R^1$ and $R^2$ are each benzyl.

4. A composition according to claim 1, in which X is oxygen.

5. A composition according to claim 1, in which n is 1, m is 0 and A is $C_1$–$C_{18}$alkyl or phenyl.

6. A composition according to claim 1, in which n and m are each 1 or n is 2 and m is 0 and A is alkylene of 2 to 6 carbon atoms, alkylidene of 2 to 6 carbon atoms, cyclohexylene, trimethyl-hexahydrobenzylene, phenylene, xylylene, 2,4-tolylene or the 4,4'-diphenylmethane radical.

7. A composition according to claim 1, in which n + m = 3 and A is

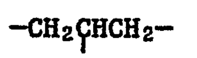 , 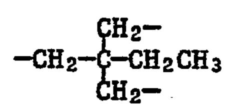

or the tris(hexylene)isocyanurate radical.

8. A composition according to claim 1, in which the organic material is a synthetic polymer.

9. A composition according to claim 8, in which the synthetic polymer is a polyolefin homopolymer or copolymer.

10. A composition according to claim 1 which also contains a metal salt of a higher fatty acid and/or a phenolic antioxidant.

11. Use of a compound of the formula I according to claim 1 for stabilizing an organic material subject to oxidative, thermal and/or actinic degradation.

12. A compound of the formula Ia

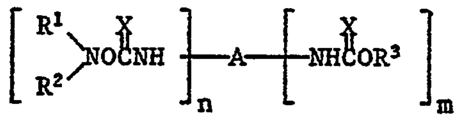 (Ia),

in which
n is 1, 2 or 3, m is 0, 1 or 2 and n + m is 2 or 3,
$R^1$ and $R^2$ independently of one another are hydrogen, $C_1$–$C_{36}$alkyl, halogen-substituted $C_1$–$C_{36}$alkyl, $C_2$–$C_6$alkoxyalkyl, $C_5$–$C_{12}$cycloalkyl, $C_7$–$C_9$aralkyl or $C_1$–$C_{36}$alkyl-substituted $C_7$–$C_9$aralkyl,
$R^3$ is $C_1$–$C_{18}$alkyl,
X is oxygen or sulfur,
A, when n + m = 2, is $C_1$–$C_{12}$alkylene, $C_2$–$C_{12}$alkylidene, $C_6$–$C_{10}$cycloalkylene, $C_1$–$C_4$alkyl-substituted hexahydrobenzylene, $C_6$–$C_{10}$arylene, xylylene or a group

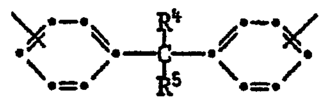

in which $R^4$ and $R^5$ independently of one another are hydrogen or $C_1$–$C_8$alkyl, and
A, when n + m = 3, is $C_3$–$C_{12}$alkanetriyl or a tris($C_2$–$C_6$alkylene)isocyanurate radical.

13. N,N′-(1,6-hexanediyl)-bis[O-(N,N-dibenzylamino)carbamate], N,N′-(4-methyl-1,3-phenylene)-di[O-(N,N-dibenzylamino)carbamate], N,N′-(4-methyl-1,3-phenylene)-O-(N,N-dibenzylamino)-O-(n-octadecyl) dicarbamate, 1-{N-[O-(N,N-dibenzylamino)carboxy]-amino}-3-{N-[O-(N,N-dibenzylamino)-carboxy]amino}-methyl-3,5,5-trimethylcyclohexane and the condensation product of N,N-dibenzylhydroxylamine and N,N′,N″-tris-(6-isocyanatohexyl)-2,4,6-trioxo-1,3,5-cyclohexyltriazine, according to claim 12.

## Revendications

1. Compositions qui comprennent une matière organique sensible aux dégradations par oxydation ou sous l'action de la chaleur et/ou d'un rayonnement actinique, avec un ou plusieurs composés de formule I ci-dessous:

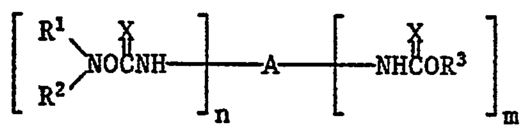 (I),

dans laquelle:
n est le nombre 1, 2 ou 3, m le nombre 0, 1 ou 2 et la somme n + m est égale à 1, 2 ou 3,
$R^1$ et $R^2$ désignent chacun, indépendamment l'un de l'autre, l'hydrogène, un alkyle en $C_1$–$C_{36}$ éventuellement halogéné, un alcoxyalkyle en $C_2$–$C_6$, un cycloalkyle en $C_5$–$C_{12}$ ou encore un aralkyle en $C_7$–$C_9$ pouvant éventuellement porter comme substituant un alkyle en $C_1$–$C_{36}$,
$R^3$ est un alkyle en $C_1$–$C_{18}$,
X l'oxygène ou le soufre, et
A, si n = 1 et m = 0, est l'hydrogène, un alkyl en $C_1$–$C_{36}$ éventuellement halogéné, un alcoxyalkyle en $C_2$–$C_6$, un cycloalkyle en $C_5$–$C_{12}$, un aryle en $C_6$–$C_{10}$, un aralkyle en $C_7$–$C_9$ avec éventuellement comme substituant un alkyle en $C_1$–$C_{36}$, ou un aryloxyalkyle ayant de 6 à 10 atomes de carbone dans la partie arylique et de 2 à 6 dans la partie alkylique, ou bien
A, si la somme n + m = 2, est un alkylène en $C_1$–$C_{12}$, un alkylidène en $C_2$–$C_{12}$, un cycloalkylène en $C_6$–$C_{10}$, un hexahydroxybenzylène avec un alkyle en $C_1$–$C_4$ comme substituant, un arylène en $C_6$–$C_{10}$ ou un xylylène ou encore un groupe

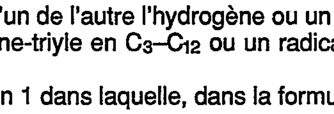

$R^4$ et $R^5$ désignant, indépendamment l'un de l'autre l'hydrogène ou un alkyle en $C_1$–$C_8$, ou enfin
A, si la somme n + m = 3, est un alcane-triyle en $C_3$–$C_{12}$ ou un radical d'isocyanurate de tris($C_2$–$C_6$-alkylène).

2. Composition selon la revendication 1 dans laquelle, dans la formule donnée, $R^1$ et $R^2$ sont chacun, in-

dépendamment l'un de l'autre, l'hydrogène, un alkyle à chaîne linéaire ou ramifiée en $C_1$–$C_{18}$ ou le groupe cyclopentyle, cyclohexyle, benzyle, α-méthylbenzyle ou α,α-diméthylbenzyle.

3. Composition selon la revendication 1, dans laquelle $R^1$ et $R^2$ sont chacun le groupe benzyle.

4. Composition selon la revendication 1, dans laquelle X est l'oxygène.

5. Composition selon la revendication 1, dans laquelle n = 1, m = 0 et A un alkyle en $C_1$–$C_{18}$ ou un phényle.

6. Composition selon la revendication 1, dans laquelle n et m sont chacun le nombre 1 ou bien n = 2 et m = 0, et A est un alkylène en $C_2$–$C_6$, un alkylidène en $C_2$–$C_6$, ou le groupe cyclohexylène, triméthyl-hexahydrobenzylène, phénylène, xylylène ou 2,4-tolylène ou encore le radical du 4,4'-diphénylméthane.

7. Composition selon la revendication 1, dans laquelle n + m = 3 et A est le radical

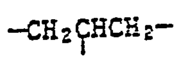 , 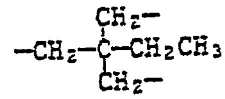

ou le radical d'isocyanurate de tris(hexylène).

8. Composition selon la revendication 1, dont la matière organique est une matière polymère synthétique.

9. Composition selon la revendication 8, dont la matière polymère synthétique est un homopolymère ou un copolymère d'oléfines.

10. Composition selon la revendication 1, qui contient également un sel de métal d'un acide gras supérieur et/ou un antioxydant phénolique.

11. Emploi des composés de formule I selon la revendication 1 pour stabiliser et protéger des matières organiques qui sont sensibles aux dégradations par oxydation ou sous l'action de la chaleur et/ou d'un rayonnement actinique.

12. Composés de formule Ia:

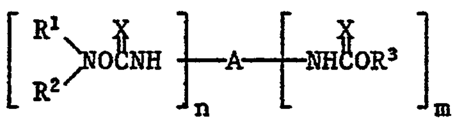 (Ia),

dans laquelle
n est 1, 2 ou 3, m est 0, 1 ou 2 et la somme n + m est égale à 2 ou 3,
$R^1$ et $R^2$ désignent chacun, indépendamment l'un de l'autre, l'hydrogène, un alkyle en $C_1$–$C_{36}$ éventuellement halogéné, un alcoxyalkyle en $C_2$–$C_6$, un cycloalkyle en $C_5$–$C_{12}$, un aralkyle en $C_7$–$C_9$ ou un aralkyle en $C_7$–$C_9$ ayant comme substituant un alkyle en $C_1$–$C_{36}$,
$R^3$ désigne un alkyle en $C_1$–$C_{18}$,
X l'oxygène ou le soufre, et
A, si n + m = 2, est un alkylène en $C_1$–$C_{12}$, un alkylidène en $C_2$–$C_{12}$, un cycloalkylène en $C_6$–$C_{10}$, un hexahydrobenzylène avec un substituant alkylique en $C_1$–$C_4$, un arylène en $C_6$–$C_{10}$, un xylylène ou un groupe

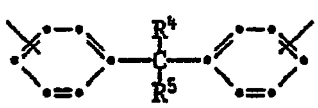

$R^4$ et $R^5$ désignant chacun, indépendamment l'un de l'autre, l'hydrogène ou un alkyle en $C_1$–$C_8$, alors que A, si n + m = 3, est un radical $C_3$–$C_{12}$-alcanetriyle ou un radical d'isocyanurate de tris($C_2$–$C_6$ alkylène).

13. N,N'-(1–6-Hexane-diyl)-bis[O-(N,N-dibenzylamino)carbamate)], N,N'-(4-Méthyl-1,3-phénylène)-di[O-(N,N-dibenzylamino)-carbamate], N,N'-(4-Méthyl-1,3-phénylène)-O-(N,N-dibenzylamino)-O-(n-octadécyl)-dicarbamate, 1-{N-[O-(N,N-dibenzylamino)-carboxy]amino}-3-{N-[O-(N,N-dibenzylamino)-carboxy]amino}-méthyl-3,5,5-triméthylcyclohexane et le produit de condensation de N,N-dibenzylhydroxylamine et N,N',N"-tris(6-isocyanatohexyl)-2,4,6-trioxo-1,3,5-cyclohexyltriazine selon la revendication 12.